# EUROPEAN PATENT SPECIFICATION

(11) **EP 0 308 514 B1**
(45) Date of publication and mention of the grant of the patent: **30.03.1994**
(21) Application number: 88902541.7
(22) Date of filing: 11.03.1988
(51) Int. Cl.: G01N 27/30, G01N 27/416

(54) **Method of fabrication of a biomicroelectrode**
Methode zur Erzeugung einer Biomikroelektrode
Méthode de fabrication d'une biomicroélectrode

(30) Priority: 13.03.1987 JP 56472/87; 03.12.1987 JP 304523/87
(43) Date of publication of application: 29.03.1989
(73) Proprietor: JAPAN, as represented by PRESIDENT OF NATIONAL REHABILITATION CENTER FOR THE DISABLED, Tokorozawa-shi, Saitama 359 (JP)
(72) Inventor: IKARIYAMA, Yoshihito, Tokorozawa-shi Saitama 359 (JP); YAMAUCHI, Shigeru, Chiyoda-ku Tokyo 102 (JP)
(74) Representative: Bühling, Gerhard, Dipl.-Chem.
(86) International application number: JP8800255
(87) International publication number: WO8807192

(56) References cited:
- No relevant documents have been disclosed.

## Description

The present invention relates to a method of fabrication of a biomicroelectrode by direct immobilization of a biologically active substance. In this technique, the biologically active substance such as enzyme can be immobilized directly in and on the surface(s) of the micro electrode, whether linking agent(s) is applied or not.

It has been known that biosensor prepared by the incorporation of enzymes, antibody or microorganisms on the surface of platinum or carbon can enable a quick and continuously measurement of various chemical substances and biologically active substances. In such preparation, the biologically active substance is applied on the surface of the electrode by preparing independently the membrane containing the biologically active substance, and adhering the membrane on the surface of the electrode. The other preparation is carried out by forming covalent bonds between the enzyme and the surface. However, while the feature of a biosensor is evaluated by reproducibility, life-period, high sensitivity and response ability, one prepared by the former method is not good with respect to reproducibility, and one prepared by the latter preparation is difficult in increasing the density of the enzymes on the surface. Further, the prior art preparation needs several steps for the formation of the bioelectrode, and in addition, it is difficult to prepare a multifunctional sensor in which several species of biologically active substances are incorporated on one sensor.

The prior art enzyme- immobilized electrode has the structure of enzyme-immobilized membrane adhered on the surface of a platinum plate. The preparation thereof may include the adhering of previously prepared enzyme-immobilized membrane on the platinum electrode, and the coating of chemically treated clean surface of the electrode with enzyme. Then, the miniaturization of the electrode is difficult. Recently, the significant technique for miniaturization is a semiconductor integration technique. In such integration technique, an enzyme electrode having a size in order of mm can be considered, but the conventional detecting method by potential measurement will result in satisfaction in sensitivity and response ability. Further, the size of the conventional electrode is difficult to be more miniatured. There has been known a quick and continuous measurement of various chemical or biological substance by a biosensor with immobilized enzyme, antibody or microorganism on the surface of platinum or carbon. In such a biosensor, the biologically active substance is immobilized by various methods for example, by adhering a membrane containing the biologically active substance to the electrode, or by coating the chemically treated surface of an electrode with enzyme or the like and forming a covalent bond between the enzyme and the surface. However, the performance of such a biosensor can be evaluated by its reproducibility, durability, sensitivity and response ability, a biosensor prepared by the former method has shortcomings in response ability, and the one prepared by the latter method is difficult in increasing the immobilization.

JP-A-61 195 346 discloses an immunosensor produced by a two-step-process wherein an electrode membrane made of poly-pyrrole or poly-thiophene is provided with a bioactive material immobilized thereon. The bioactive material such as an antigen or an antibody is immobilized by adsorption to the polymeric membrane. The polymeric membrane is produced by electrolyte polymerization of pyrrole or thiophene in the electrolyte solution; then the membrane is immersed in the solution containing the antigen or the antibody.

JP-A-56 163 447 discloses an enzyme electrode produced by a three-step-process wherein a substrate, mainly consisting of graphite, is coated with a thin - less than 1 µm - layer of platinum (first step). On this electrode a solution of an enzyme (glucose oxidase) is applied (second step). After drying the enzyme layer is crosslinked by reaction with glutaric aldehyde vapor (third step).

US-A-4 224 125 discloses an enzyme electrode (redox-electrode) wherein the enzyme and, if necessary its coenzyme, are entrapped in a polymer which mediates the an electron transfer (so called redox polymer, like hydroxymethylated polyacrylamide). The entrapping process is effected simply by mixing the polymer with the enzyme in an aqueous solution. Subsequently the redox polymer entrapping the enzyme is placed in a space at or near an electron collector.

JP-A-61-61049 discloses an oxidase electrode in which the oxidase is immobilized or fixed on the surface of a paste electrode. The carbon paste electrode is formed by mixing a carbon powder with fluid paraffine showing hydrophobic property.

The older, but postpublished EP-A 0 304 494 describes a microbioelectrode provided with a conductive fine particle layer which has been electrochemically or electrolytically deposited on the minute surface of an electroconductive material and a biologically active substance entrapped in said conductive fine particle layer by immersing said layer into a solution containing said active substance.

In any of the prior methods for the fabrication of a biosensor, several complicated steps are necessary to achieve immobilization, and further it is difficult to fabricate a multifunctional biosensor which incorporates several species of biologically active substances in one biosensor.

With the foregoing considerations in mind the present invention provides a method of fabrication of a biomicroelectrode by immobilization of a biologically active substance, which comprises the single step of electro-depositing fine particles of a conductive material from a soluble precursor of said conductive material together with said active substance on a part of the surface of an electrode substrate, said conductive material electro-deposited on said electrode substrate forming a porous conductive material layer or conductive fine particle layer incorporating said active substance entrapped therein.

The invention is advantageously developed by the measures mentioned in the dependent claims.

The present invention resides in the provision of a method of fabrication of a biosensor system to overcome the shortcomings of the prior art biosensor.

Further, the micro device using the micro electrode obtained by the process according to the present invention as a functional electrode may provide a biosensing system with high performance to measure extremely small quantity of sample. The method according to the present invention can provide a biosensing analytical system which can detect trace substance(s) ( for example, glucose ) in a micro sample(s) in a very small amount by measuring a current generated when a potential is applied in the micro sample(s). Further, the method according to the present invention may provide an analytical system and method which can detect independently of the amount of the sample(s) to be measured. The bioanalytical system obtained by the method of the present invention can use voltametry techniques, and then, can measure the sample(s) even in a stationary state. Because the bioanalytical method uses detection of active substance(s) incorporated in the electrode, it can be applied on an important enzyme such as oxidizing enzyme and dehydogenating enzyme. Further, the bioanalytical system can be used as an electrode for enzyme immunological analysis.

In the biomicroelectrode obtained by the method in accordance with the present invention a biologically active substance is unified to the fine particles of an electrically conductive material so as to fabricate a surface layer of fine particles of an electrically conductive material incorporating the biologically active substance, and then the electrode having such surface conductive layer is used as a functional electrode, and assembled together with a means for applying a potential and a means for measuring the current as generated, and then the measured current value(s) will determine the concentration of trace substance(s). Further, in the analytical method, when a given potential is applied to a functional electrode which is fabricated by unifying the biologically active substance within the surface layer of the fine particles of an electrically conductive material on the surface of the electrode, electric current is generated and measured, and then, the concentration of trace substance(s) in the very small sample can be determined from the measured value(s) of the generated current. This unifying process of incorporating the bioactive substance within the surface layer of the fine particles of an electrically conductive material is accomplished by the single step of depositing the fine particles of an electrically conductive material on the surface of the electrically conductive material, with simultaneous adsorption of the bioactive substance. The biomicroelectrode of the electrically conductive material incorporating the biologically active substance can have a very small size, and the performance similar to that of the larger bio electrode and the structure has the surface layer of the fine particles of an electrically conductive material, incoporating the immobilized biologically active substance. The biomicroelectrode obtained in accordance with the present invention can be used as a transducer element and as a bioreactor not only as a bio measurement element. Such biomicroelectrode can be refered to as a microelectrode. In other words, it has been found that when the biologically active substance immobilized microelement obtained by the method according to the invention is used, and exerted to application of the given potential, an electric current is generated and can determine the concentration of trace substance(s) in the sample(s) by the certain relation thereto of the measured current value(s).

The biomicroelectrode fabricated in accordance with the present invention has a layer of fine particles of electrically conductive material consisting preferably of platinum and the like, which incorporates the bioactive substance such as enzyme, as formed on the surface of very small plate electrode (e.g. size of 1 to 100 micrometer ). Particularly, the electrode having a surface layer of platinum black formed by electrochemical deposition of platinum is well known to evidence high catalytical activity for hydrogenation, but the incorporation of the biologically active substance in such a platinum black layer has not been known, (while there has been known immobilization of enzyme and the like in the pores of platinum plate fabricated by etching, and then binding the enzyme thereon by a crosslinking agent.) Further, it has not been known that the size of the platinum black can be controlled as in accordance with the present invention so as to incorporate the biologically active substance therein, to immobilize thereby the biologically active substance. In other words, the method of direct immobilization of the biologically active substance in accordance with the present invention does not necessarily need a chemical agent (crosslinking agent) as in a carrier binding technique, but enables the direct immobilization of the biologically active substance without any chemical treatment.

In accordance with the inventive method of fabrication of a biomicroelectrode a biologically active substance is electrically deposited directly on an electrically conductive material such as platinum, and simultaneously precipitating fine particles of an electrically conductive material (for example metal ) together with the biologically active substance by electrolysis reducing (electrolyting) metal salt. In other words, fine particles of the conductive material are being formed on a small surface of e.g. platinum, incorporating a biologically active substance in the pores among the fine particles of the conductive material. The size of the pore and the amount of the immobilized biologically active substance can be controlled (adjusted ) by current density, electrolyting period and applied potential. The function of the resulting biologically active substance immobilized electrode can be maintained for longer period. The fabricated electrode can have a thin film of polymer material such as protein or polysuccharide covered thereon and crosslinked by crosslinking agent, so as to allot the biological adaptability and long life to the electrode, and to minimize the solubility of the biologically active substance

Hereinafter, "biologically active substance" may include enzyme and antibody as a representative, and various catalyst, microorganism, proliferated microorganism, organelle, antigen, antibody and hapten. Further, in the method according to the present invention, in place of platinum, "an electrically conductive material" such as gold, rhodium, ruthenium oxide (RuO₃), carbon, palladium, and indium may be used. Any conductive material the fine particles layer of which can be formed on the surface of the electrically conductive material can be used for the electrode, unless the other trouble can be.

A polymer material used for covering additionally the surface of the biosensor made in accordance with the present invention may include proteins such as albumin, polysaccharide such as heparin. The usable crosslinking agent is preferably a crosslinking agent adapted to the used polymer material which may include glutaraldehyde adapted to albumin, and further include carbo diimido, maleate imide crosslinking agent.

Further, a mediater such as ferrocene can be incorporated in the fine particles of the biologically active substance so as to enable measurement of target material even in absence of soluble oxygen or with less soluble oxygen where oxygen has to be dissolved in the solution containing target material for the measurement. Further it enables to reduce significantly the potential necessary to operate the sensor.

The inventive method of immobilizing the biologically active substance is an extremely important technology for the development of clinical chemical analysis, and portable health check system requiring multiple detecting and multiple functions. Recently, there have been proposed various multiple biosensors using the integrated circuit technique, and the inventive method of immobilization of an enzyme and the like is important in this phase too. Further, it is evident that the biomicroelectrode, e.g. the enzyme electrode fabricated by the inventive method has high and quick response.

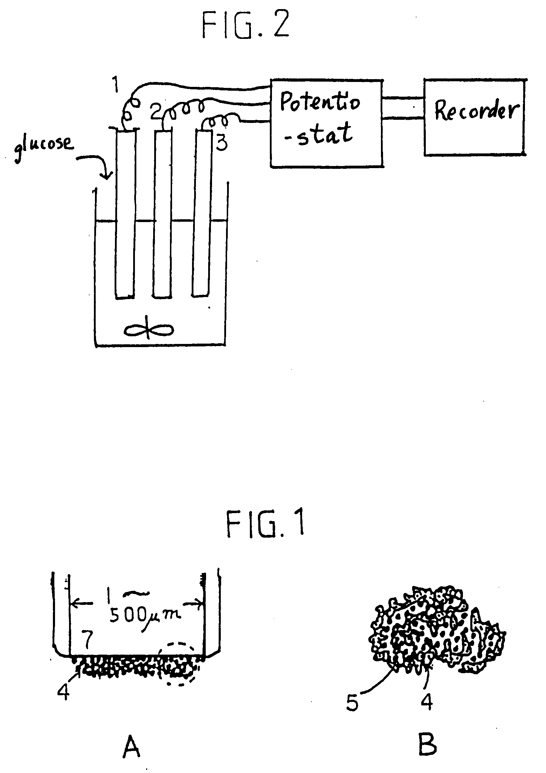

The structure of the electrically conductive material layer incorporating the immobilized biologically active substance is as shown in FIG. 1 A, B. The fine particles of the the biologically active substance are incorporated homogeneously as shown in the conductive fine particles. In other words, enzyme and the like are incorporated in a small size electrode by electrolysis depositing the conductive material in very fine particles so as to form a biologically active substance immobilized electrode.

In accordance with the present invention, the biologically active substance is immobilized in high density in a conductive material so as to afford a highly sensitive electrode in use for a biosensor. For example, the electrode having an immobilized enzyme and the like in a surface layer of platinum black of a platinum electrode has a high sensitivity when it is used as biosensor for measurement by amperemetry. Therefore, using a method of immobilizing the biologically active substance in accordance with the present invention, a biosensor can be fabricated using microelectrodes.

The size of fine particles of the biologically active substance or the size of pore can be adjusted by changing the preparation conditions, or, by controlling the reduction current, reduction period, or the reduction potential, or by controlling the concentration of lead acetate in the electrolysis solution when platinum black or gold black is formed.

As a material for electrode, gold, other noble metal, carbon such as glassy carbon, graphite carbon as well as platinum can be used for a substrate, and then, fine particulate material such as platinum black, gold black, fine particles of noble metal, or fine particles of conductive metal oxide in layer form is formed together with the biologically active substance.

A biosensing system is obtained by utilizing platinum black formation and electrochemical adsorption of enzyme thereon so as to fabricate a very small and efficient enzyme electrode biomicroelectrode), and then using the highly quick response and high sensitivity.

The biomicroelectrode having a biofunction obtained by the method according to the invention has the structure comprising a surface layer of fine particles of electrically conductive material such as platinum including the biologically active substance such as enzyme. Such utilization of platinum black for a carrier for the biologically active substance as in the element has not been known ( whereas there has been known that platinum plate is etched to form porous plate in which enzyme and the like are settled and crosslinked to their surfaces by a crosslinking agent.) Further, there has not been known that the size of platinum black fine particles can be controlled so as to incorporate the biologically active substance therein.

The fabrication of the microelectrode in accordance with the present invention uses a direct immobilization of the biologically active substance which is not the prior art carrier binding method requiring a chemical agent (crosslinking agent) and can exert direct fixation of the biologically active substance without any chemical treatment.

The method of fabricating the microelectrode used in the present invention consists of the step of depositing directly the biologically active substance on the conductive material such as the sectional face of a platinum wire, simultaneously reducing metal salt (electrolysis) to form a deposit of fine conductive particles (e.g. metal) incorporating the biologically active substance. For example, fine particles of platinum are formed on a part of the surface of platinum electrode, and the biologically active substance is simultaneously incorporated in the small pores with the fine particles so as to fabricate the biomicroelectrode. The size of the pores and the amount of the immobilized biologically active substance can be adjusted by adjusting the current density, the electrolysis period and the given potential. The function of the biologically active substance immobilized electrode can be maintained for a long period either with or without further chemical treatment.

Accordingly, the size of the microelectrode is dependent on the diameter of the used platinum wire, and then, when a very thin wire is used, the bioelectrode can be significantly smaller than that expected in the prior art. Therefore, a bioelectrode with size in micron order can be easily fabricated, and can be conveniently used for medical application e.g. intravital application, and then when it is used for analytical purpose, an apparatus with very little sample cell can be manufactured so as to enable analysis of trace amount sample, and further, quick measurement and high response ability can be easily afforded. In addition, when the electrode with larger size is fabricated, the analytical apparatus can be operated with higher output for detection.

In the fabrication of the microelectrode, the conductive material to be used as a substrate should not necessarily be the same as the conductive material to be deposited as fine particles (porous material). For example, the structure in which platinum black is deposited on the surface of graphite can be used. Further, "electrically conductive material" such as gold and rhodium can be used in place of platinum for the formation of porous conductive material, and further, any material that can form a conductive layer of fine particles on the conductive surface can be used unless other trouble can be considered.

The electrode consisting essentially of platinum black can be in various form such as in disc form, in spherical form or in tubular form. Therefore, it can satisfy any requirements based on the measurement by a biosensor, or other conditions, and then, it can be utilized for bioreactor.

Further, the microelectrode obtained by the present invention can be covered with polymeric material such as protein and polysaccharide for coating, and then, crosslinked with the cover film by crosslinking agent so as to give a biological adaptation, to have longer life, and to minimize the resolution of the biologically active substance.

"Biologically active substance" may include enzyme and antibody as a representative, and various catalyst, microorganism, proliferated microorganism, organelle, antigen, antibody and hapten.

Further, mediator such as ferrocene can be incorporated in the fine particles of the biologically active substance so as to measure a target substance in absence of dissolved oxygen or presence of little oxygen, thereby to reduce significantly the operating potential of the sensor.

The method of electro-depositing fine particles of a conductive material for preparing the biomicroelectrode can use non-electrolysis whereas the above mentioned fabrication is carried out by electrolysis deposition.

Further, the micro enzyme electrode is electrochemically treated or anodizingly treated so as to improve the selectivity of the measurement.

The analytical method can practice a miniatured bio sensing system with high speed response and high sensitivity. This will be an extremely important technique for the development of clinical analysis or portable health checking system requiring miniaturization of biosensor and multiple detecting or multiple function.

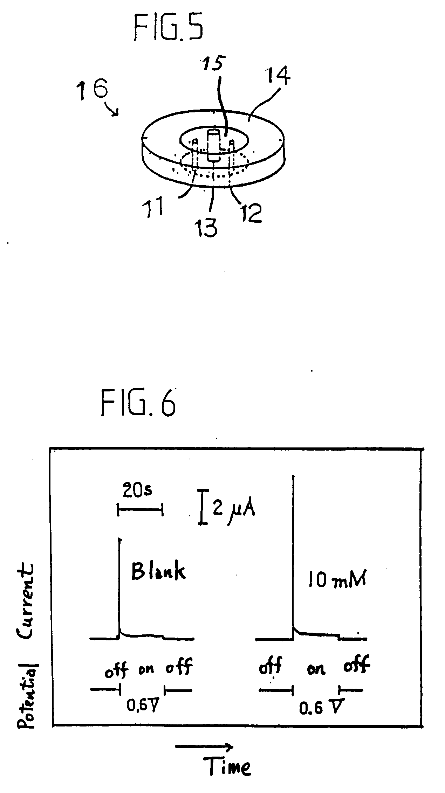

FIG. 5 shows a micro device 16 comprising a micro electrode 11 with diameter of about 1 micrometer to 500 micrometer as a functional electrode, a counter electrode 12 of platinum wire, and a reference electrode 13 of silver/silver chloride, those three electrodes fixed in the resin 15 settled in a hole of PTFE (poly tetrafluoro ethylene) resin 14. Since such micro device 16 comprises merely three metal wires as settled therein, the device can be in very small size.

When such micro device is used, it enables to measure on sample in very small amount, for example, solution in merely one micron liter. After titration of a very small amount sample, potential is applied, and the current generated thereby is detected to determine the amount of the target material in the sample.

Accordingly, the active material as generated in the electrode by the biologically active substance such as enzyme molecular can be directly measured in real time.

Various voltmetry detections can be applied to the above electrode, and then, the sample can be measured on even without stirring the sample liquid.

The inventive method of immobilizing the biologically active substance can afford the biomicroelectrode, which will provide firstly a good response ability, and highly sensitive detection, and secondly a whole incorporation of the biologically active substance(s) such as enzyme without any harm to the active substance(s) by easy and direct immobilization. Thirdly, it will provide a high density of the active substance(s) immobilized on the surface of the microelectrode to afford very quick response, and fourthly, an easily and readily immobilization of the active substance(s) such as enzyme without any chemical treatment on the very small surface of the electrode, and fifthly, a multifunctional enzyme sensor with a very small surface, in which plurality of enzymes are immobilized or settled. The electrode element can measure quickly and sensitively because it has an apparent small surface area, but, a very large surface area several thousands times than the apparent surface area of the electrode which is fabricated from the deposition of fine particles on the surface of the electrode, so as to increase the sensitivity of the element.

An analytical system obtained by the method in accordance with the present invention can provide firstly a high performance biosensing system with highly quick response and high sensitivity which enables to measure a sample without stirring the sample liquid and secondly can enable to reduce the amount of the sample to be measured, because micro enzyme electrode (i.e. biomicroelectrode), counter electrode and reference electrode can be arranged in a very small surface. Thirdly, it can detect directly an active material generated by enzyme molecules within the electrode so as to measure in real time. Fourthly, various voltametry technique can be used so as to enable detecting with enough sensitivity even without stirring the sample liquid. Fifthly, it uses an active substance(s) in the electrode, and then, important enzyme or biologically active substance(s) such as oxidizing enzyme and dehydrogenating enzyme can be used. Sixthly, it will provide an intravital biosensing system or portable biosensor.

The biomicroelectrode made in accordance with the present invention has a very large surface area of several thousands times than the apparent area of the electrode so as to function as a large electrode, thereby increasing the detection sensitivity. The inventive biosensor electrode has a fine particle layer, and the active substance(s) can permeate enough deeply in the electrode and be immobilized, and then quick response can be provided. The inventive method of immobilization of the active substance(s) provides the active substance directly immobilized or fixed in spaces among the fine particles of the electrically conductive material so as to be easy to be fabricated.

### SIMPLE DESCRIPTION OF DRAWINGS

FIG. 1 A illustrates schematically the section of structure of the biomicroelectrode prepared by the inventive method of immobilizing the biologically active substance. FIG. 1 B illustrates schematically the enlarged section of the encircled portion of FIG. 1 A, as shown by a dotted line, to show the detailed structure of fine particulate conductive material impregnated with the biologically active substance.

FIG. 2 illustrates schematically the method of measuring glucose concentration by using the biomicroelectrode in a batch system.

FIG. 3 is a graph showing the response ability of the biosensor element in the batch system measurement as shown in FIG. 2.

FIG. 4 is a graph showing the relationship of responded sensor output and glucose concentration measured by the biosensor element in the batch system measurement as shown in FIG. 2.

FIG. 5 illustrates schematically the perspective view of a micro device of a three electrode system( measuring by pulse method), using the biomicroelectrode as shown in FIGS. 1 A and 1 B.

FIG. 6 is a graph showing the current generated when a constant pulsed potential is applied to the micro device as shown in FIG. 5.

FIG. 7 is a graph showing the relationship of the generated current value and glucose concentration measured by the biosensor element as shown in FIG. 5.

FIG. 8 is a graph showing the relationship of the volume of glucose sample and peak current value as measured by the analytical system by the pulse method as shown in FIG. 5.

FIG. 9 is a graph showing detailedly the transient current response to a pulsed potential as shown in FIG. 6.

FIG. 10 is a graph showing the relationship between the glucose concentrations and the difference between the response current and the blank response current as measured after two milliseconds after the application of the pulsed potential as shown in FIG. 9.

FIG. 11 is a graph showing the relationship between the glucose sample quantity and the difference between the response current and the blank response current as measured after two milliseconds after the application of the pulsed potential as shown in FIG. 9.

FIG. 12 illustrates schematically the structure of the detecting portion of the flow injection analysis using the biomicroelectrode made in accordance with the present invention.

FIG. 13 is a graph showing the result in its response ability when the sample containing glucose is measured by using the flow injection analysis.

FIG. 14 is a graph showing the relationship of the glucose concentration and the response output, as measured by using the flow injection analysis.

FIG. 15 is a graph showing the improvement in the selectivity of the trace substance to be detected, as measured by using the oxidation treated biomicroelectrode element.

FIG. 16 is a graph showing the selectivity of the trace substance to be detected, as measured by using a biomicroelectrode which is not oxidation treated.

The structure of the biomicroelectrode, as fabricated by the inventive immobilization of the biologically active substance in accordance with the present invention in shown in FIGS. 1 A and 1 B in the sectional view and its enlarged view. There is provided micro electrode having the fine particles 4 of the electroconductive material 4, the fine particles 5 of the biologically active substance, and the electrically conductive substrate 7.

FIG. 5 shows micro device 16 comprising a micro enzyme (glucose oxidase) electrode having a micro electrode 11 of about 1 micrometer to 500 micrometer in diameter, as a functional electrode, and a counter electrode 12 of platinum wire, and a reference electrode 13 having silver / silver chloride. Those three electrodes, i.e. microelectrode 11, a counter electrode 12 and a reference electrode 13 are fixed in the resin 15 put in the hole of PTFE (poly tetrafluoro ethylene ) resin 14 and settled. Such micro device 16 has the structure having merely three metal wire fixed, and then, can have a very small size.

Therefore, the micro electrode can detect on a very small quantity of sample, for example, one microliter of the sample. After the very small amount of sample is put in, the potential is applied, and then the generated current value is detected to determine the amount of the trace substance to be detected, in the sample.

Accordingly, the device can detect directly the active substance generated in the electrode by the biologically active substance for example enzyme contained in the micro electrode. Further, the electrode can be exerted by voltametry detecting method in various form, and then, the sample can be detected even without stirring the sample liquid.

Because the electrode can detect the active subtance therein, it can be used for the bioanalysis of the important biologically active substance such as oxidized enzyme or dehydrogenated enzyme.

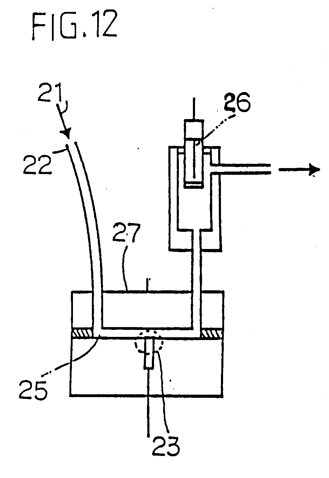

FIG. 12 illustrates in sectional view the analytical apparatus by using flow injection method. The liquid 21 containing the trace substance to be detected is charged from an inlet 22, passes through a flow having a micro electrode 23, and passes through a flow having a reference electrode 26 and a counter electrode 27 so as to detect the trace substance contained in the liquid sample 21. The biomicro electrode has a unified structure of the biologically active substance and platinum black on the face of the platinum wire, and then, the device can be fabricated in very small size, for example, the size of micrometer order in diameter can be microfabricated, and then the flow cell as shown in the drawings can be fabricated in very small size.

Accordingly, when the bio sensing system is miniatured so as to detect the very small quantity even in a small amount of the sample, when the biologically active substance immobilized electrode, for example, enzyme embodied electrode is utilized. Further, in the continuous measurement, several hundreds of samples can be treated per one minute of time.

Further, the bio analytical system obtained by using the biomicroelectrode made in accordance with the present invention can use a potentiometry measurement.

The present invention is detailedly illustrated by the following examples, but should not be interpreted for the limitation of the invention.

### EXAMPLE 1

### Fabrication of biologically active substance immobilized electrode and its feature measurement

Glucose is changed into gluconic acid and hydrogen peroxide in the presence of glucose oxidase, and then, its concentration can be determined by measuring the oxidation current due to hydrogen peroxide responding to the glucose concentration by using an electrode incorporating the enzyme with platinum electrode.

First of all, the preparation of enzyme fixed electrode was carried out, and then, the test for measuring glucose concentration by using the resulting electrode was carried out.

A platinum wire of 100 micrometer in diameter was fixed at a tube of soda glass and was polished at its end surface by alumina powder.

The immobilization of enzyme was carried out by the following two different processes.
(A) Electrolysis was carried out in a solution of sodium sulfate ( 0.2M, pH=3.2 ) containing hexachloroplatinate ( 1 mg/ml) and glucose oxidase ( 1 mg/ml ), using silver/silver chloride electrode as a reference electrode, and the prepared platinum wire as a functional electrode, by applying a constant potential ( - 0.2 V ), for ten minutes, to form a deposit of platinum black incorporating enzyme.
(B) Electrolysis was carried out in 1 ml of a solution of sodium sulfate ( pH=3.5 ) containing hexachloroplatinate (33 mg ), lead acetate ( 0.6 mg) and glucose oxidase ( 10 mg ), by applying a constant current ( - 5 microampere ), for ten minutes, to form a deposit of platinum black incorporating enzyme.

The resulting electrode was washed overnight in a 0.1 M buffer solution of phosphoric acid, and tested for the measurement of glucose concentration as follows;
The prepared electrode is easily fabricated in one step, and used for the measurement in batch system with stable and quick response. The structure of the deposit layered surface of the electrode is as shown in FIG. 1 B in sectional view, and constitutes a sensing portion.

In the preparation of the electrode as mentioned above in accordance with the present invention, a glucose oxidase ( isoelectric point = 4.2 ) charged positively was deposited on the surface of the electrode to form an electrolyted platinum, and as a result, enzyme was immobilized in the pores of platinum black fine particles.

The device of FIG. 2 has a reference electrode 1, a counter electrode 2, a functional electrode 3, electrically conductive material fine particles 4, a biologically active substance 5 and a conductive substrate 7.

Enzyme can be immobilized by any of the above two processes of fabrication of the electrode. The adjustment of fixed enzyme amount can be rather effected by the process (A) to form a uniform enzyme incorporated fine particle platinum black layer because enzyme being positively charged was deposited in a negative potential range, and the platinum black can grow at a constant potential. Then, in the process (B) wherein the deposit is formed by applying a constant current, the potential is changed to decrease gradually a current density, when the deposited platinum black layer grows, and therefore, the time for the preparation is longer, and it will be more difficult to form a homogeneous deposit.

The biomicroelectrode was tested to evaluate its response ability by measuring in a buffer solution of phosphoric acid of a measurement apparatus in batch system as shown in FIG. 2. The numeral 1 represents a reference electrode (standard electrode ), 2 is a counter electrode and 3 is a functional electrode, the glucose oxidase fixed platinum black electrode prepared in accordance with the present invention. Each of those electrodes were connected as shown in the drawings to each electrodes of potentiometeric apparatus. Where plus 0.6 volt was applied to the functional electrode 3 relatively to the reference electrode 1, the solution was agitated, and glucose was added to, and the oxidation current generated between the counter electrode and the functional electrode was measured depending on the generated hydrogen peroxide.

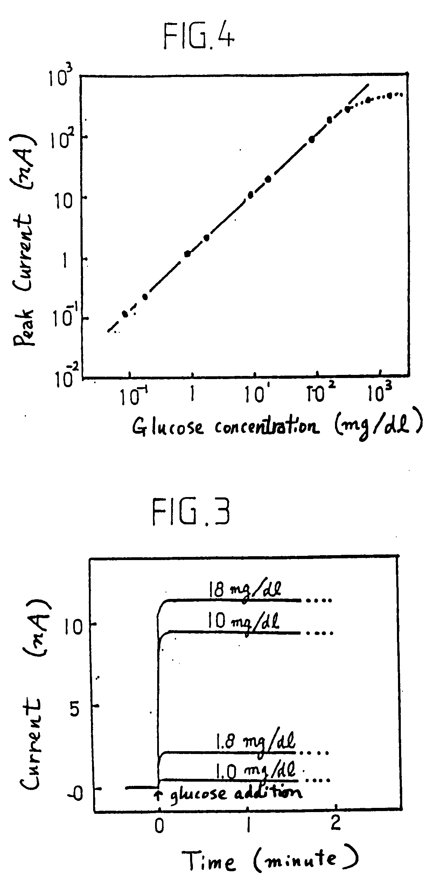

The output of the sensor of the present invention associated with the addition of glucose to the solution, that is, the change of the oxidation current generated by hydrogen peroxide shows a very quick response as shown in FIG. 3. It has been found to respond within three seconds to the rate of 100 %. The response of the sensor was very quick, and reached rapidly to the constant value. The measured result is shown in FIG. 4 showing the relation between the glucose concentration and the output of the sensor. It is evident that the biosensor can measure even the concentration of 0.1 mg/dl, and the linearity of the value and the concentration was found within the range to be measured of 0.1 mg/dl to 100 mg/dl.

It was apparent that the biosensor prepared by the immobilization of the biologically active substance in accordance with the present invention has a quick response ability and high sensitivity, which can be easily and readily fabricated.

### Example 2

### Analytical apparatus and the method thereby using the biomicro electrode made in accordance with the present invention.

A fine platinum wire of 100 micrometer in diameter, a platinum wire of 200 micrometer in diameter for a counter electrode, and a silver wire of 500 micrometer in diameter were fixed by the resin 14, and then, the surface of the fine platinum wire was polished by using alumina powder. Against such polished surface, enzyme was immobilized as follows.

The polished wire was dipped in the solution 1 ml (pH =3.5) containing 33 mg of hexachloroplatinate, 0.6 mg of lead acetate and 10 mg of glucose oxidase, and electrolysis was exerted for ten minutes, by applying a constant potential ( -0.2 V) or a constant current ( -0.5 microampere), to form a deposit of glucose oxidase immobilized platinum black.

Then, the micro device 16 using micro electrode as shown in FIG. 5, as mentioned above is using a silver wire as a silver/silver chloride reference electrode, and then, washed in a 0.1 M buffer solution of phosphoric acid overnight resulting in three electrode microdevice. FIG. 5 shows a functional electrode ( microelectrode ) 11, a counter electrode 12, a reference electrode 13, resin 15, PTFE ( poly tetrafluro ethylene ) 14 and the microdevice 16.

### Measurement of glucose concentration by using enzyme immobilized electrode.

20 Microliter of glucose sample was put in the micro device of three electrodes system as shown in FIG. 5, and the potential of 0.6 volt was applied. That is, several samples of different concentration of glucose were dropped in the device applying 0.6 volt of potential. The peak current values generated were measured and the relation of the measured current values and the glucose concentrations was investigated. The result is as shown in FIG. 6.

When 0.6 volt of potential was applied, the current as shown in FIG. 6 was generated. Where the sample did not contain glucose, the current as shown the left portion of FIG. 6 was generated. Where the sample was a buffer phosphoric acid solution containing 10 mM glucose ( 50 mM, pH=7, 0.05 mM NaCl ), the response current as shown in the right portion of FIG. 6.

As apparent from FIG. 6, the response current was generated immediately after the potential was applied, and then, the response current was rapidly reduced. This will support that the hydrogen peroxide generated by glucose oxidizing enzyme would be oxidized directly and immediately when the potential was applied. Further, it was understood that when the potential was repeatedly applied, the peak current will be at a constant value after several time cycle of application.

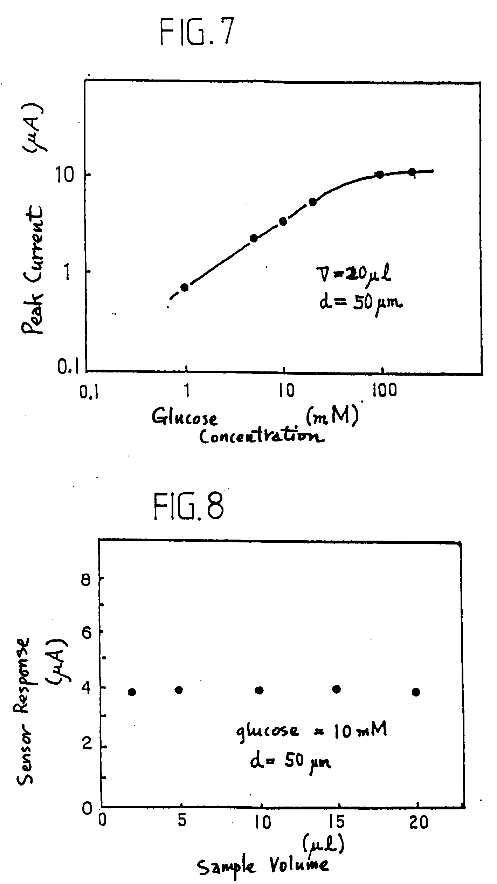

Such phenomenon was found when the potential was applied in a buffer solution of phosphoric acid in a similar way. The peak current values were measured of the samples containing a given concentration of glucose, and the peak current generated in a buffer solution not containing glucose was measured ( blank value ). The difference values from the peak value to the peak value at not -containing glucose solution were calculated, and the relation of the difference values and the given glucose concentrations was investigated. The result is shown in FIG. 7 wherein the volume (V) of the sample solution is 20µl and the size (d) of the electrode is 50µm. That is, where the glucose concentration changes from 1 mM to 100 mM, the measured curve showing the relation to the peak current is found as in FIG. 7.

Therefore, it is supported that the enzyme analytical system can measure the concentration of the trace substance in real time even in a sample without being stirred in a very small amount.

### Test of sample quantity dependence as measured by the device.

2, 5, 10, 15, and 20 microliter of the glucose standard sample containing 10 mM of glucose were respectively picked up for each tests and dropped in the micro biosensor, and the currents generated when the potential of 0 .6 volt was applied were measured for each test. The result is shown in FIG. 8 with abbreviations as explained above It was found that the peak current generated is independent of the quantity of the sample.

### Selective measurement of concentration by Transient response measurement in pulse voltmetry

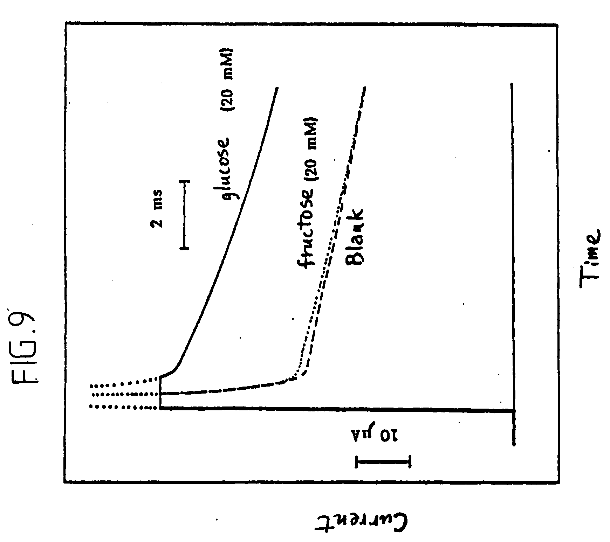

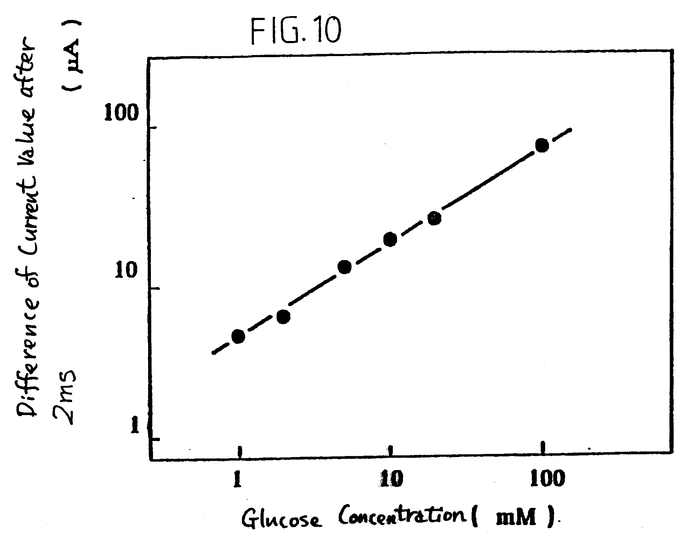

The initial stage of the response of the sensor was recorded by a transient memory when the potential of 0.6 volt was applied to the enzyme electrode for the detailed measurement of the pulse shown in the right portion of FIG. 6. The result is sown FIG. 9. There is found a significant difference of the initial response to the glucose containing buffer solution from the initial response to the blank sample ( phosphoric acid buffer ) and to the fructose-containing phosphoric acid buffer. Therefore, the difference of the the current value at two milliseconds time after the application of the potential from the glucose containing sample to the blank sample is plotted against the concentration of glucose as shown in FIG. 10. The linearity of the output to the concentration of glucose was found in the range of 1 mM to 100 mM.

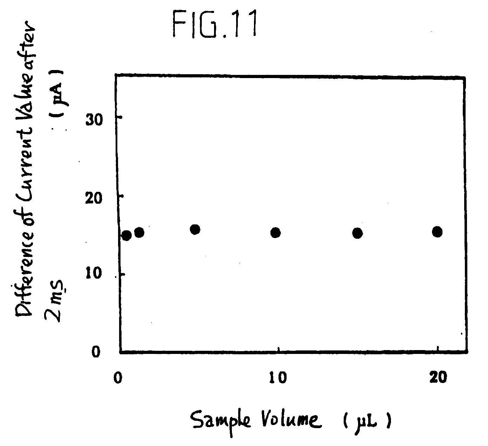

Further, the relation of the sample amount to the output of the sensor is investigated where the concentration of glucose is constant. The measured result is shown in FIG. 11. It was confirmed that the output of the sensor is not dependent on the amount of the samples.

It is noted that the analytical system obtained in accordance with the method in accordance with the present invention can give information on the whole sample under stationary state by detecting on a portion of the sample in the measurement.

Further, the analytical system does not need the agitation of the sample, and therefore, can be used as an intravital biosensing system or as a portable biosensor.

The bioanalytical system can be used in a countercurrent vessel or in a batch form as well as in a stationary vessel, and can detect with high speed response and quick measurement and high sensitivity.

### Example 3

### Analytical apparatus and method by flow injection using the micro electrode

There will be illustrated a method of fabricating an enzyme embodied electrode ( biomicro electrode ) to be used for analysis. First of all, the end surface of small platinum wire of 100 micrometer in diameter was polished by alumina powder to be a micro platinum electrode. Electrolysis was carried out for ten minutes in one mililiter of a solution (pH=3.5) containing hexachloroplatinate ( 33 mg ), lead acetate ( 0.6 mg) and glucose oxidase ( 10 mg ) by applying a constant potential ( - 0.2 V ) or a constant current ( -5 microampere ) to form a deposit of platinum black incorporating glucose oxidase. The prepared deposit of platinum black incorporating glucose oxidase was about several micrometer in thickness.

Next, the resulting microelectrode was used as a functional electrode, and assembled into the microdevice having the structure as shown in FIG. 12. In a flow injection measurement, the liquid 21 to be measured flows from an inlet 22, and passes the passage 25 having a microelectrode 23. A silver/silver chloride electrode was used as a reference electrode 26, and a stainless steel block electrode was used as a counter electrode 27.

### Measurement of glucose concentration

A liquid to be measured was put into a flow injection measurement system as shown in FIG. 12 by a single pump (ER 8711 type) manufactured by Erma Inc. A stainless steel block electrode was used as a counter electrode, and a silver/silver chloride electrode was used as a reference electrode. Potential of 0.6 volt was applied to the microelectrode, and hydrogen peroxide being generated was oxidized. 0.1 M phosphoric acid buffer solution ( pH=6.8 ) was used as a mobile phase, and several kinds of samples containing glucose of different concentration were detected. The flow rate varied in the range of 0.4 ml/minute to 1.8 ml/minute.

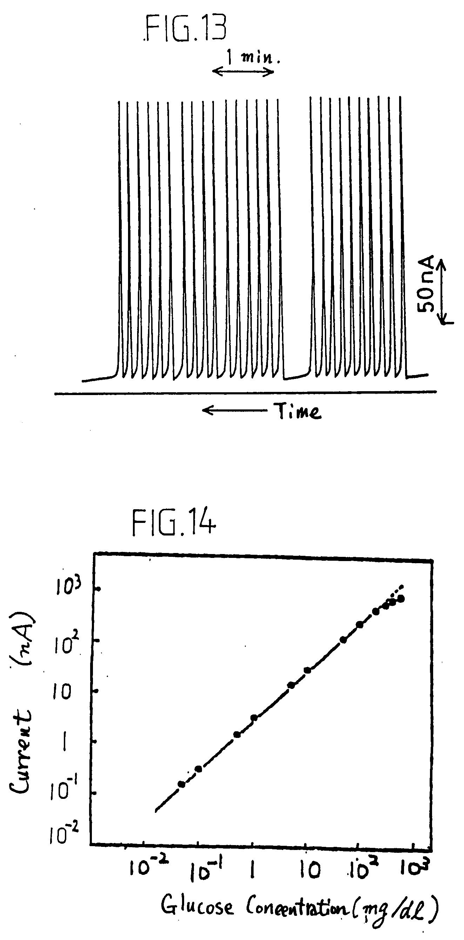

Using a microelectrode having a deposit of glucose oxidase incorporated platinum black formed on the end surface of a platinum wire of 100 micrometer in diameter, the response to the intermittent charge of glucose solution ( 10 mM ) was measured and recorded. The result is shown in FIG. 13. As shown in the drawings, the current generated reached the peak three seconds after charge of the sample and returned to the original level within ten seconds.

While seven samples were charged one after another within one minute, the detected current shows complete independence of the various peaks corresponding to each charge of the samples. It was found that one sample could be detected within about nine seconds. The time period necessary for one sample to complete the measurement, that is, the response time for measurement of glucose is closely dependent on the size of the microelectrode. It is apparent that when the diameter of the microelectrode is smaller, the time period necessary to measure is shorter. The high speed measurement can be accomplished by reducing the diameter of the microelectrode.

Next, the time period necessary to measure the concentration of glucose and the peak current were investigated changing the flow rate of the mobile phase solution. As a result, it was found that the time necessary to measure the concentration of glucose was shorter, when the flow rate was higher, and that the value of the peak current was lower, when the flow rate was higher.

The relation of the glucose concentration and the peak current is shown in FIG. 14. It was apparent from FIG. 14 that the linearity of the peak current and the concentration of glucose is established in the range of 50 µM to 20 mM.

### Example 4

### Result of anodic polarization

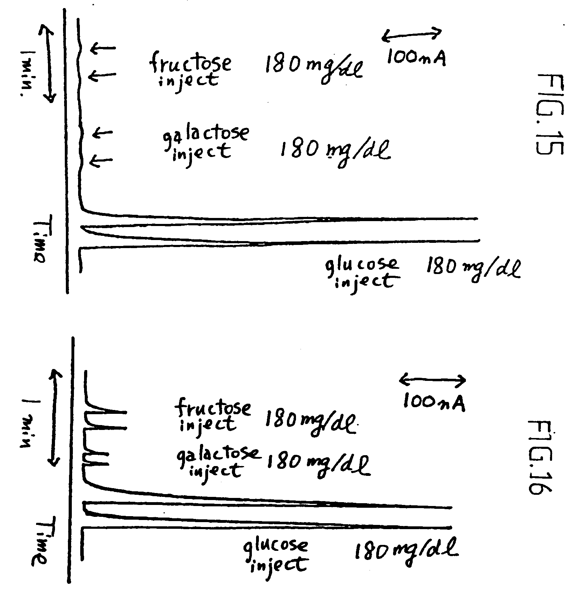

A platinum wire of 100 micrometer in diameter was sealed on a block of acryl resin, and the surface of the block was polished by alumina abrasives having particles of 30 micrometer to 0.05 micrometer in size. Next, it was dipped in a solution of hexachloroplatinate of 3 % containing 500 ppm of lead acetate and 10 % of glucose oxidase, and electrolysis was carried out to form a deposit of platinum fine particles on the smooth end surface of the platinum wire. This electrolysis was for five minutes carried out at - 0.08 volt. Six platinum fine particle electrode were fabricated, and then, among them, three of them were treated by oxidation (anodic) treatment, and three of them were not treated. The treatment was carried out by dipping the resulting enzyme embodied porous electrode, and applying 1.0 volt to said enzyme electrode for ten minutes. Then, the resulting glucose oxidase embodied electrode was washed overnight with a 0.1 M phosphoric buffer solution. The resulting glucose oxidase embodied electrode was assembled as a functional electrode in the flow measurement apparatus as shown in FIG . 11. The sample solutions containing various kinds of saccharide were put in the measurement system in flow injection as shown in FIG. 11 by using a single pump (SPY 2502 U) manufactured by Nippon Seimitu Co. so as to measure on the oxidized electrode in the samples containing various saccharides. A stainless steel block electrode was used as a counter electrode. 0.1 M phosphoric acid buffer solution was used as a mobile phase solution. Potential of 0.6 volt was applied to the glucose oxidase embodied electrode. The response curve was measured when the oxidized (anodic polarization ) glucose oxidase embodied electrode was used, and the resulting curve is shown in FIG. 15. Then, the response curve was measured by using the untreated glucose oxidase embodied electrode, and the resulting curve is shown in FIG. 16.

Ordinarily, the saccharides such as glucose, galactose and fructose can not be oxidized at the potential of 0.6 volt. However, in this experiment, glucose can be oxidized by the action of catalytic glucose oxidase because of the glucose oxidase embodied electrode, and the oxidizing current can be detected corresponding to the amount of the glucose therein. Then, the other saccharides can not be oxidized because it is not effected by the glucose oxidase, and the current change may not be caused even if the solution containing the other saccharides were injected in the cell solution. However, the not - treated (not- anodic polarization) glucose oxidase embodied electrode caused a current due to the injection of galactose and fructose. Contrarily, there was not found the current change based on the injection of the other saccharide than glucose when the oxidation treated ( anodic polarization) glucose oxidase embodied electrode was used.

The process of immobilizing of a biologically active substance in accordance with the present invention, a biomicroelectrode prepared thereby, and an analytical method using the said biomicroelectrode can be utilized for a variety of measurements and a measurement apparatus using a biologically active substance, and a biomicroelectrode which can function as a transducer or a reacting element, are adapted for the measurement and the analytical apparatus with extremely small size, extremely quick response and high sensitivity.

## Claims

1. A method of fabrication of a biomicroelectrode by immobilization of a biologically active substance, which comprises the single step of electro-depositing fine particles (4) of a conductive material from a soluble precursor of said conductive material together with said active substance on a part of the surface of an electrode substrate (7), said conductive material electro-deposited on said electrode substrate (7) forming a porous conductive material layer or conductive fine particle layer incorporating said active substance entrapped therein.

2. A method according to claim 1, wherein said conductive material (4) is selected from the group of platinum, gold, rhodium, palladium, indium and ruthenium oxide (RuO₃).

3. A method according to claim 1 or 2, wherein said conductive material layer is treated by anodic oxidation.

4. A method according to either one of claims 1 to 3, wherein the biomicroelectrode is further stabilized by a crosslinking agent.

5. A method according to either one of claims 1 to 4, wherein a polymeric film is formed on the surface of said conductive material layer, so as to prevent said active substance from leaking out.

## Patentansprüche

1. Verfahren zur Herstellung einer Biomikroelelektrode mittels Immobilisierens einer biologisch aktiven Substanz, welches den einzigen Schritt einer Elektro-Abscheidung von feinen Teilchen (4) eines leitenden Materials aus einem löslichen Vorläufer desselben gleichzeitig mit der aktiven Substanz auf einem Teil der Oberfläche eines Elektrodensubstrats (7) umfasst, wobei das leitfähige Material auf dem Elektrodensubstrat (7) unter Bildung einer die darin eingefangene aktive Substanz inkorporierenden, porösen, leitfähigen Materialschicht oder leitfähigen, feinpartikulären Schicht gebildet wird.

2. Verfahren nach Anspruch 1, wobei das leitfähige Material (4) aus Platin, Gold, Rhodium, Palladium, Indium oder Rutheniumoxid (RuO₃) ausgewählt wird.

3. Verfahren nach Anspruch 1 oder 2, wobei die leitfähige Materialschicht mittels anodischer Oxidation behandelt wird.

4. Verfahren nach einem der Ansprüche 1 bis 3, wobei die Biomikroelektrode mittels eines quervernetzenden Agens desweiteren stabilisiert wird.

5. Verfahren nach einem der Ansprüche 1 bis 4, wobei ein Polymerfilm auf der Oberfläche der leitfähigen Materialschicht gebildet wird, so daß die aktive Substanz am Auslaufen gehindert wird.

## Revendications

1. Un procédé de fabrication d'une biomicroélectrode par immobilisation d'une substance biologiquement active qui comprend la seule étape d'électrodéposition de fines particules (4) d'une matière conductrice provenant d'un précurseur soluble de ladite matière conductrice en même temps que de ladite substance active sur une partie de la surface d'un substrat (17) d'électrode, ladite matière conductrice déposée par électrodéposition sur ledit substrat (17) d'électrode formant une couche de matière conductrice poreuse ou une couche de particules fines conductrice dans laquelle est incluse ladite substance active.

2. Un procédé selon la revendciatino 1, dans lequel ladite matière conductrice (14) est choisie dans le groupe comprenant l'oxyde de ruthenium (RuO₃), indium, palladium, rhodium, or et platine.

3. Un procédé selon la revendication 1 ou 2, dans lequel ladite couche de matière conductrice est traitée par oxydation anodique.

4. Un procédé selon l'une quelconque des revendications 1 à 3, dans lequel la biomicroélectrode est en outre stabilisée par un agent de réticulation.

5. Un procédé selon l'une quelconque des revendications 1 à 4, dans lequel une pellicule polymère est formée sur la surface de la couche de matière conductrice de façon à empêcher que ladite substance active ne s'échappe.
